(19)

Europäisches
Patentamt
European
Patent Office
Office européen
des brevets

(11)  **EP 3 714 692 A1**

(12)  **EUROPEAN PATENT APPLICATION**

(43) Date of publication:
**30.09.2020  Bulletin 2020/40**

(21) Application number: **20166237.6**

(22) Date of filing: **27.03.2020**

(51) Int Cl.:
**A01N 25/08** (2006.01)     **C04B 35/14** (2006.01)
**C04B 35/624** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.03.2019  TW 108111403**

(71) Applicant: **Kaohsiung Medical University
807 Kaohsiung City (TW)**

(72) Inventors:
• **SHIH, Chi-Jen
  807 Kaohsiung City (TW)**

• **LEE, Chung-Lin
  807 Kaohsiung City (TW)**
• **YANG-WANG, Yuan-Ting
  807 Kaohsiung City (TW)**
• **CHIANG, Yu-Ching
  807 Kaohsiung City (TW)**
• **CHEN, Yu-Hsuan
  807 Kaohsiung City (TW)**

(74) Representative: **Patentanwaltskanzlei WILHELM
& BECK
Prinzenstraße 13
80639 München (DE)**

(54)  **ANTIBACTERIAL COLLOID AND METHOD FOR MANUFACTURING THE SAME**

(57)     An antibacterial colloid, comprising: a plurality of metal nanoparticles. wherein the plurality of metal nanoparticles have an average particle diameter less than 10 nm; a plurality of metal ions, wherein the plurality of metal ions have a concentration greater than 20 ppm; and a medium, wherein the medium comprises a protein component containing at least a functional group for reduction, wherein the antibacterial colloid is free from nitrate ions.

EP 3 714 692 A1

Printed by Jouve, 75001 PARIS (FR)

**Description**

The present invention relates to an antibacterial colloid and a method of manufacturing the same. More particularly, the present invention relates to an antibacterial colloid manufactured through a ceramic substrate having a hierarchically meso-macroporous structure acting with a medium comprising at least one protein component to make its slow released metal ions reduce into a plurality of metal nanoparticles and a method of manufacturing the same.

In recent years, the emerging silver nanoparticles (AgNP) preparation methods have mainly been based on green medium activation synthesis methods. Most of the synthetic methods use silver nitrate as a precursor of silver ions ($Ag^+$) and reduce $Ag^+$ by microorganisms, plant extracts, etc. and use them as stabilizers for AgNP, in which the nucleation rate and growth rate of AgNP cannot be controlled. The synthesized AgNP are not stable, and they are also prone to agglomeration, deposition and precipitation. The particle diameter of the synthesized AgNP is larger (average particle diameter -50 nm) and the size distribution is uneven. However, AgNP capable of effectively inhibiting multiple drug-resistant organisms (MDRO) should have a particle diameter of less than 10 nm, which is the technical bottleneck when using these synthetic methods to inhibit MDRO. On the other hand, the AgNP prepared by these synthetic methods will retain nitrate ions ($NO_3^-$), and the nitrate ions are easily bonded to reductive cations or metal ions in a solution, which will cause redox reactions during thermal decomposition, and have thermal instability characteristics. In terms of toxicity, studies on animal toxicity *in vivo* indicate that silver nitrate is much more toxic than AgNP. In addition, nitrate ions are contaminants present in the environment, and recent studies have indicated that they may be toxic to human reproductive systems.

Mesoporous silica-based materials are typical amorphous phase solids with a high specific surface area, low toxicity, good encapsulation and slow release ability. The silver-containing mesoporous silica-based materials synthesized in previous studies use deionized water or simulated body fluid as a slow release medium to form $Ag^+$, but the obtained $Ag^+$ concentration is only about 5 ppm. This $Ag^+$ concentration can only inhibit general non-resistant strains (*Escherichia coli*, *Staphylococcus aureus*, and *Pseudomonas aeruginosa*) and the stability is low. After a period of time, silver particles are precipitated, showing a non-uniform phase. Most of the previous studies do not address the toxicity of the silver-containing mesoporous silica-based materials to cells.

Based on the above, the applicant proposes the present invention "antibacterial colloid and method for manufacturing the same" in view of the disadvantages of the prior art to improve the abovementioned disadvantages.

In accordance with one aspect of the present invention, a method for manufacturing an antibacterial colloid is disclosed. The method comprises steps of providing and mixing raw materials or precursors thereof constituting a ceramic substrate, a metal raw material or a precursor thereof, and a template surfactant of forming a mesoporous structure to form a mixture, wherein the raw materials or the precursors thereof constituting the ceramic substrate comprise at least silicon and oxygen; synthesizing the mixture to form an initial gel by a sol-gel technique; providing a three-dimensional configuration template, wherein the three-dimensional configuration template has a macroporous structure; immersing the three-dimensional configuration template in the initial gel at least one time; forming the ceramic substrate by performing a heat treatment at or above 400°C on the immersed three-dimensional configuration template, wherein the ceramic substrate has a hierarchically meso-macroporous structure having a plurality of first metal nanoparticles confined therein, and the plurality of first metal nanoparticles have a positive slow release effect; providing a medium, wherein the medium comprises a protein component containing at least a functional group for reduction; mixing and oscillating the medium with the ceramic substrate to cause the plurality of first metal nanoparticles to release a plurality of metal ions therefrom by the positive slow release effect; and reducing a part of the plurality of metal ions to nucleate and grow by the protein component, wherein the part of the plurality of metal ions grow up to form a plurality of second metal nanoparticles; wherein the antibacterial colloid comprises the medium, the plurality of second metal nanoparticles having an average particle diameter less than 10 nm, and the plurality of metal ions having a concentration greater than 20 ppm, and the antibacterial colloid is free from nitrate ions.

In accordance with another aspect of the present invention, an antibacterial colloid in a system containing microorganisms is disclosed. The antibacterial colloid comprises a plurality of metal nanoparticles, wherein the plurality of metal nanoparticles have an average particle diameter less than 10 nm; a plurality of metal ions, wherein the plurality of metal ions have a concentration greater than 20 ppm; and a medium, wherein the medium comprises a protein component containing at least a functional group for reduction, wherein: the antibacterial colloid is free from nitrate ions; and the microorganisms have a first value A1 of the colony forming unit (CFU) in a first state, and a second value A2 of the CFU in a second state after the antibacterial colloid is added to the system for a specific period of time where (A1-A2)/A1 is greater than or equal to 0.5.

In accordance with a further aspect of the present invention, an antibacterial colloid is disclosed. The antibacterial colloid comprises a plurality of metal nanoparticles, wherein the plurality of metal nanoparticles have an average particle diameter less than 10 nm; a plurality of metal ions, wherein the plurality of metal ions have a concentration greater than 20 ppm; and a medium, wherein the medium comprises a protein component containing at least a functional group for reduction, wherein the antibacterial colloid is free from nitrate ions.

The above objectives and advantages of the present disclosure will become more readily apparent to those ordinarily skilled in the art after reviewing the following detailed descriptions and accompanying drawings, in which:

Fig. 1 shows a flow chart of a method for manufacturing a silver-containing antibacterial colloid according to the first embodiment of the present invention;

Fig. 2 shows a transmission electron microscope image of the silver-containing antibacterial colloid according to the first embodiment of the present invention;

Fig. 3 shows a measurement result of the silver-containing antibacterial colloid according to the first embodiment of the present invention by an ultraviolet spectrophotometer;

Fig. 4 shows a measurement result of the silver-containing antibacterial colloid according to the first embodiment of the present invention by an inductively coupled plasma mass spectrometer;

Fig. 5 shows a time-killing curve plot of a time-killing curve test of *Staphylococcus aureus* carried out by adding the silver-containing antibacterial colloid according to the first embodiment of the present invention in a liquid medium;

Fig. 6 shows a time-killing curve plot of a time-killing curve test of *Pseudomonas aeruginosa* carried out by adding the silver-containing antibacterial colloid according to the first embodiment of the present invention in a liquid medium;

Fig. 7 shows a time-killing curve plot of a time-killing curve test of Methicillin-resistant *Staphylococcus aureus* (MRSA 33592) carried out by adding the silver-containing antibacterial colloid according to the first embodiment of the present invention in a liquid medium;

Fig. 8 shows a time-killing curve plot of a time-killing curve test of Methicillin-resistant *Staphylococcus aureus* (MRSA 49476) carried out by adding the silver-containing antibacterial colloid according to the first embodiment of the present invention in a liquid medium;

Fig. 9 shows a time-killing curve plot of a time-killing curve test of Methicillin-resistant *Staphylococcus aureus* (VISA 700698) carried out by adding the silver-containing antibacterial colloid according to the first embodiment of the present invention in a liquid medium;

Fig. 10 shows a time-killing curve plot of a time-killing curve test of Methicillin-resistant *Staphylococcus aureus* (VISA 700699) carried out by adding the silver-containing antibacterial colloid according to the first embodiment of the present invention in a liquid medium; and

Fig. 11 shows a time-killing curve plot of a time-killing curve test of *Klebsiella pneumoniae* carried out by adding the silver-containing antibacterial colloid according to the first embodiment of the present invention in a liquid medium;

The present invention will now be described more specifically with reference to the following embodiments. It is to be noted that the following descriptions of preferred embodiments of this invention are presented herein for the purposes of illustration and description only; they are not intended to be exhaustive or to be limited to the precise form disclosed.

The invention provides a method for manufacturing an antibacterial colloid, comprising: providing and mixing raw materials or precursors thereof constituting a ceramic substrate, a metal raw material or a precursor thereof, and a template surfactant of forming a mesoporous structure to form a mixture, wherein the raw materials or the precursors thereof constituting the ceramic substrate comprise at least silicon and oxygen; synthesizing the mixture to form an initial gel by a sol-gel technique; providing a three-dimensional configuration template, wherein the three-dimensional configuration template has a macroporous structure; immersing the three-dimensional configuration template in the initial gel at least one time; forming the ceramic substrate by performing a heat treatment at or above 400°C on the immersed three-dimensional configuration template, wherein the ceramic substrate has a hierarchically meso-macroporous structure having a plurality of first metal nanoparticles confined therein, and the plurality of first metal nanoparticles have a positive slow release effect; providing a medium, wherein the medium comprises a protein component containing at least a functional group for reduction; mixing and oscillating the medium with the ceramic substrate to cause the plurality of first metal nanoparticles to release a plurality of metal ions therefrom by the positive slow release effect; and reducing a part of the plurality of metal ions to nucleate and grow by the protein component, wherein the part of the plurality of metal ions grow up to form a plurality of second metal nanoparticles; wherein the antibacterial colloid comprises the medium, the plurality of second metal nanoparticles having an average particle diameter less than 10 nm, more preferably about 2 nm to 5 nm, and the plurality of metal ions having a concentration greater than 20 ppm, and the antibacterial colloid is free from nitrate ions.

The invention provides a method for manufacturing an antibacterial colloid, wherein one of the raw materials or precursors thereof constituting the ceramic substrate may be Tetraethyl orthosilicate, the metal raw material or the precursor thereof may be silver nitrate, gold hydrogen nitrate, zinc nitrate hexahydrate, cupric nitrate trihydrate, iron trinitrate nonahydrate or strontium nitrate, and the template surfactant of forming the mesoporous structure may be a thermoreversible hydrogel (Pluronic F-127).

The invention provides a method for manufacturing an antibacterial colloid, wherein the sol-gel technique is a low-temperature wet chemical synthesis method for manufacturing ceramics and glass, and the technique comprises the conversion of a liquid phase (sol) to a solid phase (gel) of a system. The reactants have a series of hydrolysis reactions and polymerization reactions to form a colloidal suspension.

The invention provides a method for manufacturing an antibacterial colloid, wherein the three-dimensional configuration template may be a porous organism or a synthetic porous body. The porous organism may be a natural sponge, and the synthetic porous body may be a polyurethane foam or a polylactic acid macroporous structure.

The invention provides a method for manufacturing an antibacterial colloid, wherein the hierarchically meso-macroporous structure comprises a plurality of macropores and a wall having a plurality of arranged mesopores, and the plurality of macropores are separated by the wall. The wall is formed from the raw materials or the precursors thereof constituting the ceramic substrate, and the plurality of first metal nanoparticles are formed from the metal raw material or the precursor thereof. The template surfactant of forming the mesoporous structure and the immersed three-dimensional configuration template are removed during the heat treatment, the macroporous structure provides channels for removing the template surfactant of forming the mesoporous structure and the immersed three-dimensional configuration template. The nitrate ions contained in the metal raw material or the precursor thereof are also removed during the heating treatment.

The invention provides a method for manufacturing an antibacterial colloid, wherein the functional group for reduction may be S-H or N-H. The protein component may be casein. The casein may be obtained from animal milk or a plant extract. The casein may have a concentration ranging from 10 g/L to 30 g/L. A condition of oscillating may be shaking at 160 rpm for 24 hours at 35 °C.

The invention provides a method for manufacturing an antibacterial colloid, which further comprises a step of providing a filter to filtrate the antibacterial colloid to remove the ceramic substrate and impurities.

The invention provides a method for manufacturing an antibacterial colloid, wherein the antibacterial colloid has a property of inhibiting the growth of a microorganism or killing a microorganism. The microorganism may be a bacterium, a virus, a fungus or a protozoan, and the bacterium may be one selected from a group consisting of *Staphylococcus aureus*, *Pseudomonas aeruginosa*, Methicillin-resistant *staphylococcus aureus*, *Klebsiella pneumoniae* and a combination thereof.

The invention provides a method for manufacturing an antibacterial colloid, wherein when a total quantity of the raw materials or the precursors thereof constituting the ceramic substrate is $M_1$ mole, a quantity of the silicon included in the raw materials or the precursors thereof constituting the ceramic substrate is $M_{Si}$ mole, a quantity of the metal raw material or the precursor thereof is $M_{metal}$ mole, the $M_{Si}$ is at least 70% of the $M_1$, and the $M_{metal}$ is less than or equal to 10% of the $M_1$. Preferably, the $M_{metal}$ is 1% of the $M_1$.

The invention provides an antibacterial colloid in a system containing microorganisms, comprising: a plurality of metal nanoparticles, wherein the plurality of metal nanoparticles have an average particle diameter less than 10 nm; a plurality of metal ions, wherein the plurality of metal ions have a concentration greater than 20 ppm; and a medium, wherein the medium comprises a protein component containing at least a functional group for reduction, wherein: the antibacterial colloid is free from nitrate ions; and the microorganisms have a first value A1 of the colony forming unit (CFU) in a first state, and a second value A2 of the CFU in a second state after the antibacterial colloid is added to the system for a specific period of time where (A1-A2)/A1 is greater than or equal to 0.5.

The invention provides an antibacterial colloid in a system containing microorganisms, wherein the microorganisms may be bacteria, viruses, fungi or protozoa, and the bacteria may be one selected from a group consisting of *Staphylococcus aureus*, *Pseudomonas aeruginosa*, Methicillin-resistant *staphylococcus aureus*, *Klebsiella pneumoniae* and a combination thereof.

The invention provides an antibacterial colloid in a system containing microorganisms, wherein the system may be a cell, a biological tissue, an organ, a cosmetic, a medicine, a medical appliance, or a biomaterial.

The present invention provides a system comprising microorganisms, adding a bioactivator and an antibacterial colloid in the system, after a specific period of time, an antimicrobial effect produces, and the system has a fractional inhibitory concentration (FIC) index. The antibacterial colloid comprises a plurality of metal nanoparticles, wherein the plurality of metal nanoparticles have an average particle diameter less than 10 nm; a plurality of metal ions, wherein the plurality of metal ions have a concentration greater than 20 ppm; and a medium, wherein the medium comprises a protein component containing at least a functional group for reduction, wherein the antibacterial colloid is free from nitrate ions, and the FIC index is less than or equal to 0.5.

The present invention provides a system comprising microorganisms, wherein the bioactivator may be Gentamicin.

The present invention provides a system comprising microorganisms, wherein the antibacterial colloid has a property of inhibiting the growth of a microorganism or killing a microorganism. The microorganism may be a bacterium, a virus, a fungus or a protozoan, and the bacterium may be one selected from a group consisting of *Staphylococcus aureus, Pseudomonas aeruginosa,* Methicillin-resistant *staphylococcus aureus*, *Klebsiella pneumoniae* and a combination thereof. The bacterium may best be *Klebsiella pneumoniae*.

The present invention provides a system comprising microorganisms, wherein the system is a cell, a biological tissue, an organ, a cosmetic, a medicine, a medical appliance, or a biomaterial.

The invention provides an antibacterial colloid, comprising: a plurality of metal nanoparticles, wherein the plurality of metal nanoparticles have an average particle diameter less than 10 nm; a plurality of metal ions, wherein the plurality of metal ions have a concentration greater than 20 ppm; and a medium, wherein the medium comprises a protein component containing at least a functional group for reduction, wherein the antibacterial colloid is free from nitrate ions.

The invention provides an antibacterial colloid, wherein more preferably, the plurality of metal ions have a concentration greater than 40 ppm.

The invention provides an antibacterial colloid, which further comprises a ceramic substrate.

The invention provides an antibacterial colloid, wherein the protein component may be obtained from an animal or a plant. The functional group for reduction may be S-H or N-H. The protein component may be casein. The casein may be obtained from animal milk or a plant extract. The protein may have a concentration ranging from 10 g/L to 30 g/L.

The invention provides an antibacterial colloid, wherein the antibacterial colloid has a property of inhibiting the growth of a microorganism or killing a microorganism. The microorganism may be a bacterium, a virus, a fungus or a protozoan, and the bacterium may be one selected from a group consisting of *Staphylococcus aureus*, *Pseudomonas aeruginosa*, Methicillin-resistant *staphylococcus aureus*, *Klebsiella pneumoniae* and a combination thereof.

The "antibacterial" described in the present invention includes "bacteriostatic" and "sterilization".

The industries to which the present invention can be applied include, but are not limited to, the cosmetics industry, the dental materials industry, the biomedical materials industry, and the biomedical pharmaceutical industry.

The products to which the present invention can be applied include, but are not limited to, various cosmetics, antibacterial wound dressings, antibacterial wound dressings for drug-resistant bacteria, bone filling materials, antibacterial bone filling materials, anti-odor oral gel, anti-odor oral mouthwash, anti-odor oral spray, dental patches, dental fillings, antibacterial dental fillings, antibacterial and anti-sensitive toothpastes, pet care oral sprays, pet care toothpastes and pet oral odor sprays.

In the prior art, silver nanoparticles (AgNP) are prepared by using $AgNO_3$ as a silver ions source, and the silver ions are completely dissociated in the solution, and are not slowly released into the solution, and the nucleation rate and growth rate cannot be automatically stopped, resulting in uneven size distribution and poor stability, and is prone to agglomeration, deposition and precipitation. In the present invention, the processes of reducing silver ions into silver atoms, nucleating and growing into silver nanoparticles are carried out in a medium comprising a protein component, and the silver-containing ceramic substrate of the present invention can slowly release silver ions. In the manufacturing method, the protein component plays the role of a reducing agent and a stabilizer, and has a function of promoting the formation of silver ions and limiting the growth of silver nanoparticles, and does not cause particle agglomeration or Ostwald ripening, and a green synthetic silver nanoparticle colloid with a uniform diameter of 10 nm or less can be obtained. In addition, the protein component contained in the antibacterial colloid of the present invention has a reducing functional group, which can enhance the release effect of silver ions, thereby releasing a larger amount of silver ions.

On the other hand, there is a step of the heat treatment in the manufacturing method of the present invention, and nitrate ions are removed in this step. As mentioned above, nitrate ions have thermal instability and animal toxicity, and may be toxic to human reproductive systems. The method for manufacturing an antibacterial colloid of the present invention can remove nitrate ions and thus has the advantages of stability and safety.

First Embodiment

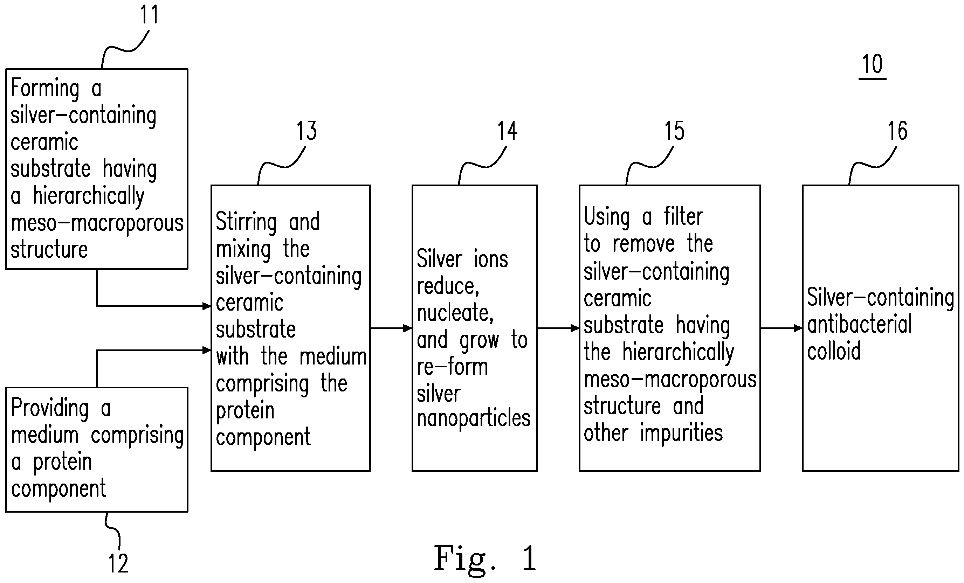

The present embodiment provides a silver-containing antibacterial colloid, a method for manufacturing the same, and a system comprising the same. In order to make the description of the embodiment more detailed and complete, Figures 1-11 can be referred. The present invention discloses an antibacterial colloid comprising a plurality of silver nanoparticles having an average particle diameter less than 10 nm, a plurality of silver ions having a concentration greater than 20 ppm, and a medium comprising at least one protein component. If the colloid is added to a system containing microorganisms, the plurality of silver nanoparticles and the plurality of silver ions can destroy the microbial structure by destroying microbial cell walls or forming reactive oxygen species (ROS), thereby achieving the effect of inhibiting the growth of microorganisms or killing microorganisms.

Please refer to Fig. 1. The present embodiment provides a method of manufacturing a silver-containing antibacterial colloid, and its process (10) includes first forming a silver-containing ceramic substrate (MMCP-Ag) having a hierarchically meso-macroporous structure (11) as a silver ions ($Ag^+$) source, then forming a colloid by a protein activation method. The steps of forming the silver-containing ceramic substrate having the hierarchically meso-macroporous structure (11) include: providing and mixing raw materials or precursors thereof constituting the ceramic substrate; a silver raw material or a precursor thereof and a template surfactant of forming a mesoporous structure to form a mixture, wherein the raw materials or the precursors thereof constituting the ceramic substrate comprise at least silicon and oxygen; synthesizing the mixture by a sol-gel technique to form an initial gel; providing a three-dimensional configuration template having a macroporous structure; immersing the three-dimensional configuration template in the initial gel at least one time; and performing a heat treatment at or above 400°C on the immersed three-dimensional configuration template to remove the three-dimensional configuration template and the template surfactant of forming the mesoporous structure. One of the raw materials or the precursors thereof constituting the ceramic substrate is Tetraethyl orthosilicate; the silver raw material or the precursor thereof may be silver nitrate; the template surfactant of forming the mesoporous structure is a thermoreversible hydrogel (Pluronic F-127); the three-dimensional configuration template having the macroporous structure is a natural sponge of a porous organism or a synthetic porous body of a porous organism, such as a polyurethane foam or polylactic acid body with macroporous structure formed by a 3-D printing technique. When a total quantity of the raw materials or the precursors thereof constituting the ceramic substrate is $M_1$ mole, a quantity of the silicon included in the raw materials or the precursors thereof constituting the ceramic substrate is $M_{Si}$ mole, a quantity of the silver raw material or the precursor is $M_{metal}$ mole, the $M_{Si}$ is at least 70% of the $M_1$, and the $M_{metal}$ is less than or equal to 10% of the $M_1$. Preferably, the $M_{metal}$ is 1% of the $M_1$. The silver-containing ceramic substrate having the hierarchically meso-macroporous structure comprises a plurality of macropores and a wall having a plurality of arranged mesopores, and the plurality of macropores are separated by the wall. The wall is formed from the raw materials or the precursors thereof, and the plurality of silver nanoparticles are formed from the silver raw material or the precursor thereof and confined in the hierarchically meso-macroporous structure and has a positive slow release effect. The positive slow release effect of the silver nanoparticles is that the silver nanoparticles positively release a concentration of at least 2 ppm of its silver ions in a hydrophilic medium at room temperature for at least 24 hours. The hydrophilic medium is one selected from a group consisting of biological fluids, aqueous solutions, alcohol, human blood, deionized water, microbial culture media, and simulated body fluids. The raw materials or the precursors thereof constituting the ceramic substrate further comprises phosphorus, calcium or a combination thereof. Of course, various raw materials or the precursors and silver raw material or the precursor thereof suitably for the ceramic substrate having the hierarchically meso-macroporous structure are within the scope of the present invention, and are not limited thereto. The steps of the protein activation method are providing a medium comprising a protein component (12), wherein the protein component contains at least one functional group for reduction, such as S-H, N-H. The present embodiment uses a medium comprising 10 g/L to 30 g/L casein, wherein the casein is from an animal milk or a plant extract. Stirring and mixing the silver-containing ceramic substrate with the medium comprising the protein component (13) by mixing the two and shaking at 35 °C, 160 rpm for 24 hours to promote the silver ions released from the silver-containing ceramic substrate having the hierarchically meso-macroporous structure reduce, nucleate, and grow to re-form silver nanoparticles (14), finally a silver-containing antibacterial colloid comprising silver nanoparticles having an average particle diameter less than 10 nm and silver ions having a concentration greater than 20 ppm (up to 68 ppm) (16) is prepared. In another embodiment, the prepared antibacterial colloid can be filtrated using a filter to remove the silver-containing ceramic substrate having the hierarchically meso-macroporous structure and other impurities (15). In another embodiment, the medium comprising the protein component can be a medium containing a *Morinda citrifolia* leaf extract. Of course, various media containing a functional group for reduction suitable for protein activation are within the scope of the present invention and are not limited thereto. A flowchart of the manufacturing method can be referred to Fig. 1. The method for manufacturing an antibacterial colloid comprising silver nanoparticles proposed in the present embodiment is different from the conventional technique, and thus the manufactured antibacterial colloid is free from nitrate ions.

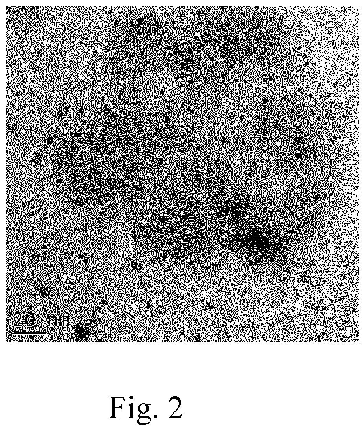

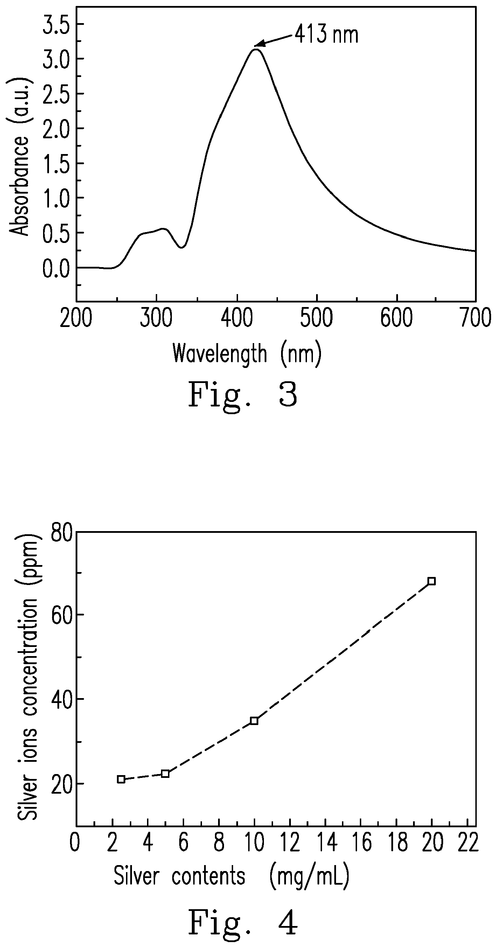

Fig. 2 shows the transmission electron microscope image of the silver-containing antibacterial colloid manufactured in the present embodiment, and the silver nanoparticles comprised therein have a particle diameter ranging from about 2 nm to 5 nm, and the average particle diameter is about 4 nm and the distribution is uniform. Fig. 3 shows the measurement result of the manufactured silver-containing antibacterial colloid according to the present embodiment by an ultraviolet spectrophotometer, which shows an absorption peak of a surface plasma resonance characteristic of the silver nanoparticles (AgNP) at a wavelength of 413 nm. It is verified that the manufacturing method proposed in the present embodiment can successfully synthesize AgNP.

Another embodiment uses a silver-containing ceramic substrate having a hierarchically meso-macroporous structure (SCNS) of different silver contents (2.5 mg/mL, 5 mg/mL, 10 mg/mL and 20 mg/mL) as a silver ions ($Ag^+$) source, which is activated by a medium comprising casein and forms a silver-containing antibacterial colloid. The inductively coupled plasma mass spectrometry (ICP-MS) is used for measurement and analysis, and the results are shown in Fig. 4. As can be seen from Fig. 4, the released silver ions concentration is 22.2 ppm, 22.5 ppm, 35.1 ppm, and 68.1 ppm, respectively. That is, the higher the silver content of SCNS, the better the effect of releasing silver ions. This silver ions concentration is 4-13 times the released silver ions limit concentration (5 ppm) disclosed in the current published literature about the silver encapsulated bioglass dissolution method.

In another embodiment, 1 mol% of silver is added to the raw materials or precursors thereof constituting a ceramic substrate having a hierarchically meso-macroporous structure such that the Si: Ca: P: Ag molar ratio of the ceramic substrate is 80: 15: 5: 1. A silver-containing ceramic substrate having a hierarchically meso-macroporous structure (SCNS) is used as a silver ions ($Ag^+$) source, which is activated by a medium comprising casein and forms a silver-containing antibacterial colloid (Casein-SCNS). The minimum inhibitory concentration (MIC) against different microorganisms of the silver-containing antibacterial colloid is measured by a time-killing curve test. The silver-containing antibacterial colloid (Casein-SCNS) formed in the present embodiment is added or not added in an environment or system containing a hydrophilic medium and microorganisms (such as liquid medium). After being cultured at 37°C for a specific period of time, each group of test solution is formed. The number of microorganisms of each group of the test solution is measured; wherein the turbidity of a certain amount of the test solution is measured and converted to obtain the number of microorganisms. The turbidity of the test solution is measured by an absorbance value (OD value) at a wavelength of 600 nm by an ELISA reader. Taking time as the horizontal axis and absorbance ($OD_{600}$) as the vertical axis, the growth curve of the test microorganisms in this environment or system is plotted. The test microorganisms may be bacteria, viruses, fungi or protozoa, wherein the bacteria may be, for example, a Methicillin-resistant *staphylococcus aureus*, a *Staphylococcus aureus*, *Pseudomonas aeruginosa*, *Escherichia coli*, *Aggregatibacter actinomycetemcomitans*, *Candida albicans*, *Klebsiella pneumoniae* and *Enterococcus faecalis*. The fungi may be, for example, *Aspergillus niger*. The condition of the liquid medium is that the ratio of the weight (mg) of the silver-containing antibacterial colloid (Casein-SCNS) extract to the volume (ml) of each specific culture medium, such as tryptic soy broth (TSB), is 5, 10, and 20. It is placed in a 37°C incubator for 24 hours at 160 rpm, and centrifuged at 3000 rpm for 5 minutes, the supernatant is then aspirated, which is the extract of the test sample. The test microorganisms are thawed and inoculated on each specific culture medium (agar), and then cultured in each specific temperature incubator for a specific period of time. The strains are scraped with sterile cotton swabs and inoculated into each sterile liquid medium. The test bacterial solution is measured by a turbidity meter, and its concentration is adjusted to about $1.5 \times 10^8$ CFU/ml (colony-forming units per milliliter). The solution is added to a 96-well microtiter plate in which the extract of the test sample is prepared, and the concentration of the bacterial solution in each well is about $5 \times 10^5$ CFU/ml after the inoculation. The bacterial solution is cultured at 37 °C, and the absorbance is measured by a spectrophotometry once every hour to 24 hours, and the time-killing curve of the time-killing curve test is plotted to find the minimum inhibitory concentration (MIC) of the silver-containing antibacterial colloid of the present embodiment on different microorganisms. The minimum inhibitory concentration (MIC) refers to the minimum concentration at which the growth of microorganisms can be inhibited and observed after 24 hours of cultivation. The control group condition is that a liquid medium containing each of the different test strains is cultured for 24 hours without adding the respective test solutions containing the silver-containing antibacterial colloid.

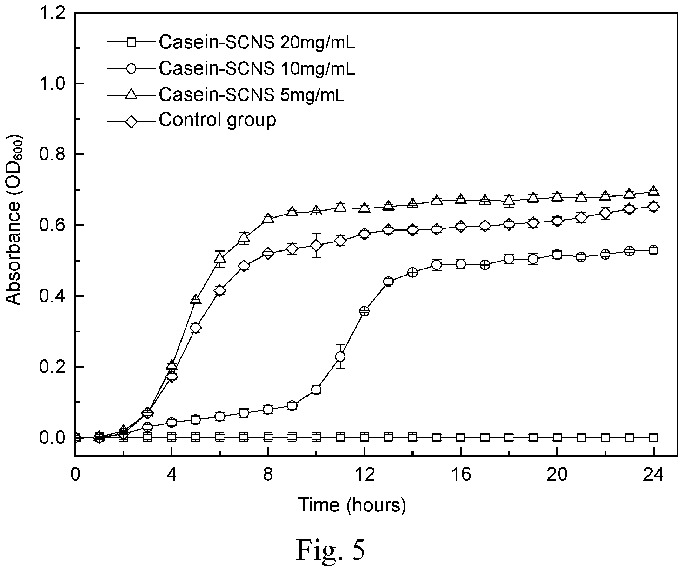

The time-killing curve test results of the silver-containing antibacterial colloid (Casein-SCNS) formed in the present embodiment against *Staphylococcus aureus*, *Pseudomonas aeruginosa*, Methicillin-resistant *Staphylococcus aureus* (MRSA 33592, MRSA 49476, VISA 700698 and VISA 700699), and *Klebsiella pneumoniae* (KP) are shown in Figs. 5-11.

1. Time-killing curve test of *Staphylococcus aureus*:

Fig. 5 shows a time-killing curve plot of the time-killing curve test of *Staphylococcus aureus* carried out by adding the silver-containing antibacterial colloid (Casein-SCNS) according to the first embodiment of the present invention in a liquid medium. The condition of the liquid medium is that the ratio of the weight (mg) of the silver-containing antibacterial colloid extract to the volume (ml) of the specific culture medium (ml) is 5, 10 and 20, respectively. It can be seen from Fig. 5 that the concentration of the silver-containing antibacterial colloid (Casein-SCNS) extract at 20 mg/mL has a good effect of inhibiting bacterial growth. The concentration of silver-containing antibacterial colloid (Casein-SCNS) extract at 10 mg/mL has a limited effect of inhibiting bacterial growth, and *Staphylococcus aureus* grows in the 10th hour. Therefore, the minimum inhibitory concentration (MIC) is estimated to be between 10 mg/mL and 20 mg/mL. The concentration of silver-containing antibacterial colloid (Casein-SCNS) extract at 5 mg/mL does not inhibit bacterial growth.

2. Time-killing curve test of *Pseudomonas aeruginosa*:

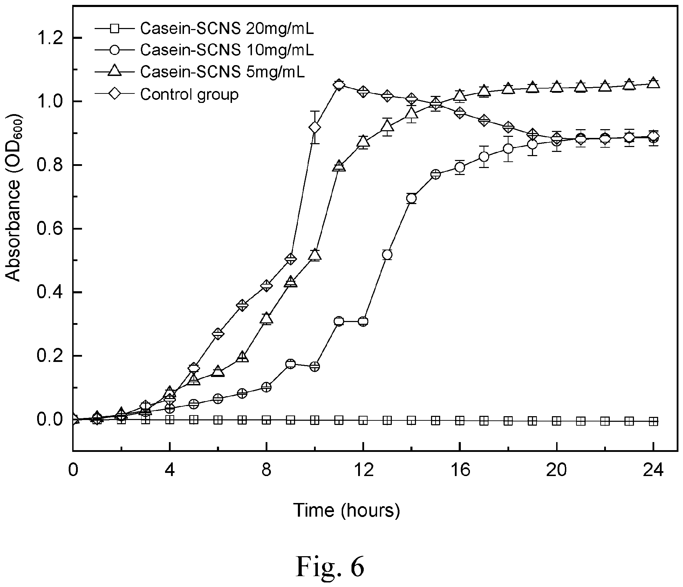

Fig. 6 shows a time-killing curve plot of the time-killing curve test of the *Pseudomonas aeruginosa* carried out by adding the silver-containing antibacterial colloid (Casein-SCNS) according to the first embodiment of the present invention in a liquid medium. The condition of the liquid medium is that the ratio of the weight (mg) of the silver-containing antibacterial colloid extract to the volume (ml) of the specific culture medium (ml) is 5, 10 and 20, respectively. It can be seen from Fig. 6 that the concentration of the silver-containing antibacterial colloid (Casein-SCNS) extract at 20 mg/mL has a good effect of inhibiting bacterial growth. The concentration of silver-containing antibacterial colloid (Casein-SCNS) extract at 10 mg/mL has a limited effect of inhibiting bacterial growth, and *Pseudomonas aeruginosa* grows in the 8th hour. Therefore, the minimum inhibitory concentration (MIC) is estimated to be between 10 mg/mL and 20 mg/mL. The concentration of silver-containing antibacterial colloid (Casein-SCNS) extract at 5 mg/mL does not inhibit bacterial growth.

3. Time-killing curve test of Methicillin-resistant *Staphylococcus aureus* (MRSA 33592):

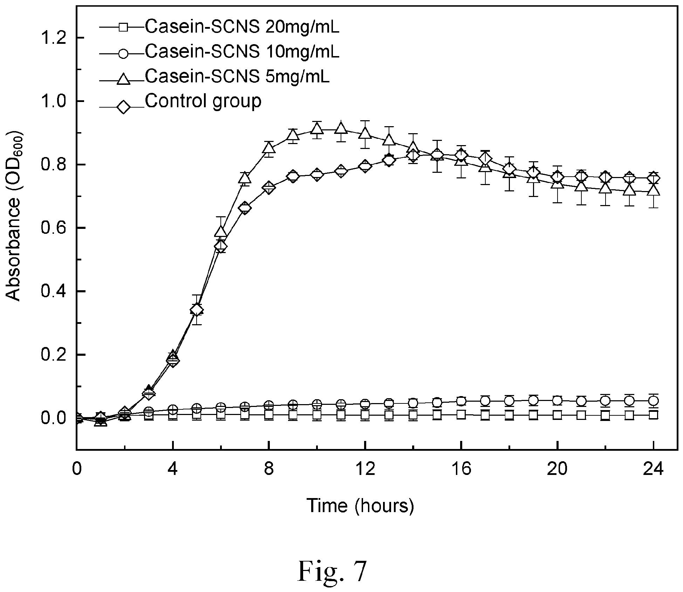

Fig. 7 shows a time-killing curve plot of the time-killing curve test of the Methicillin-resistant *Staphylococcus*

*aureus* (MRSA 33592) carried out by adding the silver-containing antibacterial colloid (Casein-SCNS) according to the first embodiment of the present invention in a liquid medium. The condition of the liquid medium is that the ratio of the weight (mg) of the silver-containing antibacterial colloid extract to the volume (ml) of the specific culture medium (ml) is 5, 10 and 20, respectively. It can be seen from Fig. 7 that the concentration of the silver-containing antibacterial colloid (Casein-SCNS) extract at 10 mg/mL and 20 mg/mL has a good effect of inhibiting bacterial growth. The concentration of silver-containing antibacterial colloid (Casein-SCNS) extract at 5 mg/mL has a limited effect of inhibiting bacterial growth, and Methicillin-resistant *Staphylococcus aureus* (MRSA 33592) grows in the 3th hour. Therefore, the minimum inhibitory concentration (MIC) is estimated to be between 5 mg/mL and 10 mg/mL.

4. Time-killing curve test of Methicillin-resistant *Staphylococcus aureus s* (MRSA 49476):

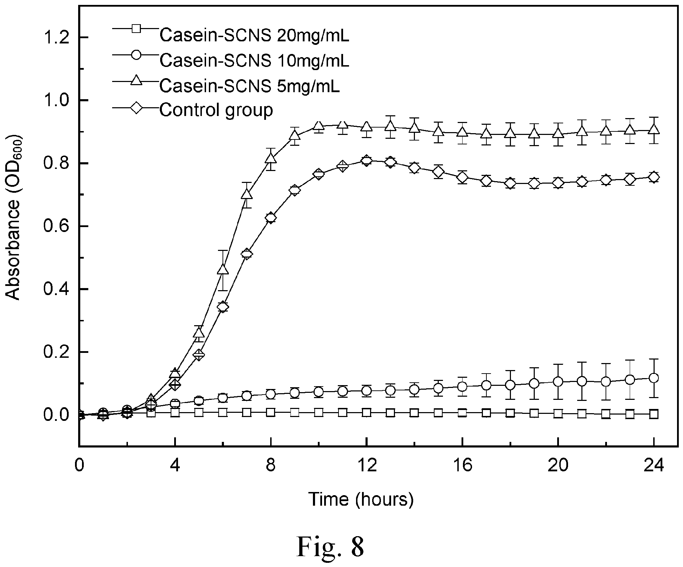

Fig. 8 shows a time-killing curve plot of the time-killing curve test of the Methicillin-resistant *Staphylococcus aureus* (MRSA 49476) carried out by adding the silver-containing antibacterial colloid (Casein-SCNS) according to the first embodiment of the present invention in a liquid medium. The condition of the liquid medium is that the ratio of the weight (mg) of the silver-containing antibacterial colloid extract to the volume (ml) of the specific culture medium (ml) is 5, 10 and 20, respectively. It can be seen from Fig. 8 that the concentration of the silver-containing antibacterial colloid (Casein-SCNS) extract at 10 mg/mL and 20 mg/mL has a good effect of inhibiting bacterial growth. The concentration of silver-containing antibacterial colloid (Casein-SCNS) extract at 5 mg/mL has a limited effect of inhibiting bacterial growth, and Methicillin-resistant *Staphylococcus aureus* (MRSA 49476) grows in the 4th hour. Therefore, the minimum inhibitory concentration (MIC) is estimated to be between 5 mg/mL and 10 mg/mL.

5. Time-killing curve test of Methicillin-resistant *Staphylococcus aureus* (VISA 700698):

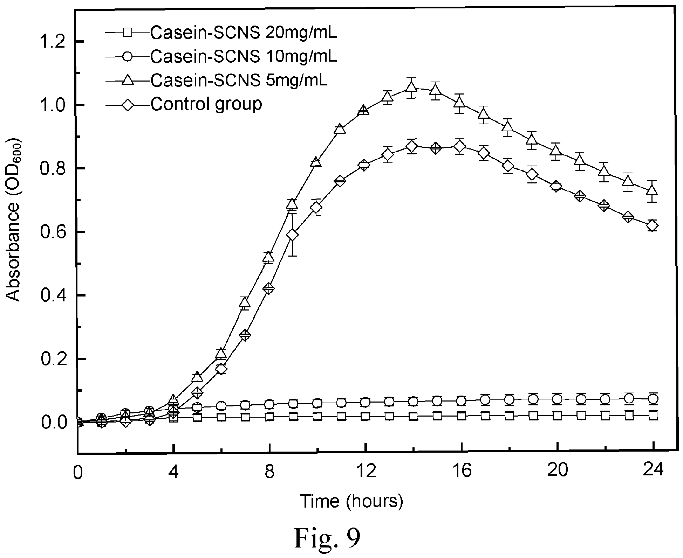

Fig. 9 shows a time-killing curve plot of the time-killing curve test of the Methicillin-resistant *Staphylococcus aureus* (VISA 700698) carried out by adding the silver-containing antibacterial colloid (Casein-SCNS) according to the first embodiment of the present invention in a liquid medium. The condition of the liquid medium is that the ratio of the weight (mg) of the silver-containing antibacterial colloid extract to the volume (ml) of the specific culture medium (ml) is 5, 10 and 20, respectively. It can be seen from Fig. 9 that the concentration of the silver-containing antibacterial colloid (Casein-SCNS) extract at 10 mg/mL and 20 mg/mL has a good effect of inhibiting bacterial growth. The concentration of silver-containing antibacterial colloid (Casein-SCNS) extract at 5 mg/mL has a limited effect of inhibiting bacterial growth, and Methicillin-resistant *Staphylococcus aureus* (VISA 700698) grows in the 5th hour. Therefore, the minimum inhibitory concentration (MIC) is estimated to be between 5 mg/mL and 10 mg/mL.

6. Time-killing curve test of Methicillin-resistant *Staphylococcus aureus* (VISA 700699):

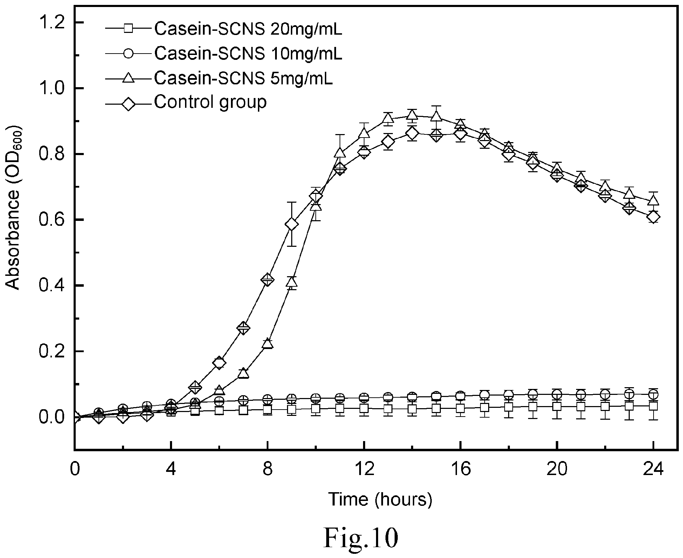

Fig. 10 shows a time-killing curve plot of the time-killing curve test of the Methicillin-resistant *Staphylococcus aureus* (VISA 700699) carried out by adding the silver-containing antibacterial colloid (Casein-SCNS) according to the first embodiment of the present invention in a liquid medium. The condition of the liquid medium is that the ratio of the weight (mg) of the silver-containing antibacterial colloid extract to the volume (ml) of the specific culture medium (ml) is 5, 10 and 20, respectively. It can be seen from Fig. 10 that the concentration of the silver-containing antibacterial colloid (Casein-SCNS) extract at 10 mg/mL and 20 mg/mL has a good effect of inhibiting bacterial growth. The concentration of silver-containing antibacterial colloid (Casein-SCNS) extract at 5 mg/mL has a limited effect of inhibiting bacterial growth, and Methicillin-resistant *Staphylococcus aureus* (VISA 700699) grows in the 7th hour. Therefore, the minimum inhibitory concentration (MIC) is estimated to be between 5 mg/mL and 10 mg/mL.

7. Time-killing curve test of *Klebsiella pneumoniae* (KP):

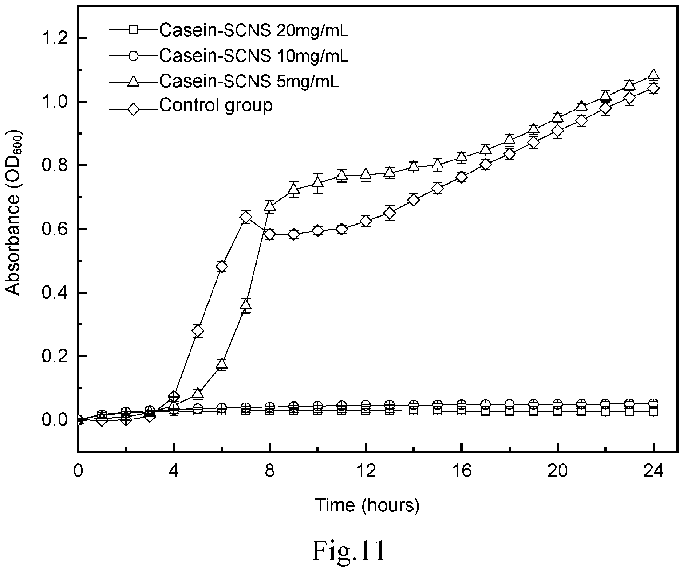

Fig. 11 shows a time-killing curve plot of the time-killing curve test of the *Klebsiella pneumoniae* carried out by adding the silver-containing antibacterial colloid (Casein-SCNS) according to the first embodiment of the present invention in a liquid medium. The condition of the liquid medium is that the ratio of the weight (mg) of the silver-containing antibacterial colloid extract to the volume (ml) of the specific culture medium (ml) is 5, 10 and 20, respectively. It can be seen from Fig. 11 that the concentration of the silver-containing antibacterial colloid (Casein-SCNS) extract at 10 mg/mL and 20 mg/mL has a good effect of inhibiting bacterial growth. The concentration of silver-containing antibacterial colloid (Casein-SCNS) extract at 5 mg/mL has a limited effect of inhibiting bacterial growth, and *Klebsiella pneumoniae* grows in the 6th hour. Therefore, the minimum inhibitory concentration (MIC) is estimated to be between 5 mg/mL and 10 mg/mL.

Conclusion: The silver-containing antibacterial colloid (Casein-SCNS) of the first embodiment of the present invention has the effect of inhibiting the growth of the above-mentioned test microorganisms, and is particularly effective for Methicillin-resistant *Staphylococcus aureus* and *Klebsiella pneumonia*. The reason is that the silver-containing antibacterial colloid (Casein-SCNS) comprises silver nanoparticles having an average particle diameter less than 10 nm and silver ions having a concentration greater than 20 ppm. These silver nanoparticles and free silver ions can destroy the microbial structure by destroying microbial cell walls or forming reactive oxidizing substances, thereby achieving the effect of inhibiting the growth of microorganisms or killing microorganisms.

Second Embodiment

The present embodiment provides a silver-containing antibacterial colloid, a method of manufacturing the same, and a system comprising the same. In order to make the description of the embodiment more detailed and complete, Tables 1-2 can be referred to. The present invention discloses an antibacterial colloid comprising a plurality of silver nanoparticles, wherein the plurality of silver nanoparticles have an average particle size less than 10 nm; a plurality of silver ions, wherein the plurality of silver ions have a concentration greater than 20 ppm; and a medium, wherein the medium comprises at least one protein component. The method for manufacturing a silver-containing antibacterial colloid of the present embodiment is the same as the first Embodiment.

The synergistic bacteriostatic test is carried out by using the silver-containing antibacterial colloid (green mediated AgNP inhibitor) manufactured in the present embodiment and a bioactivator (using Gentamicin as an example). The fractional inhibitory concentration (FIC) index is used as a reference index for synergy. Synergy is the phenomenon in which two or more substances are mixed with each other, and the overall effect is greater than the advantage of each substance alone or less than the disadvantage of each substance alone. The synergistic bacteriostatic test is carried out by using a checkerboard test with $K.$ $pneumoniae$ 700623 and $K.$ $pneumoniae$ BAA-1705 as experimental strains, respectively. The bioactivator Gentamicin is first diluted to 160 $\mu$g.mL$^{-1}$ to 640 $\mu$g.mL$^{-1}$, and 50 $\mu$L of Gentamicin is added laterally to the 96-well microplate in a two-fold serial dilution using microbial culture medium (MHB). In the two-fold serial dilutions, the concentration of Gentamicin used for $K.$ $pneumoniae$ 700623 ranging from 1.25 $\mu$g.mL$^{-1}$ to 40 $\mu$g.mL$^{-1}$, and the concentration of Gentamicin used for $K.$ $pneumoniae$ BAA-1705 ranged from 5 $\mu$g.mL$^{-1}$ to 160 $\mu$g.mL$^{-1}$. The green mediated AgNP inhibitor extract extracted with TSB medium is used, and the above procedure is repeated to dilute the concentration of the green mediated AgNP inhibitor extract to 10 mg.mL$^{-1}$. 50 $\mu$L of green mediated AgNP inhibitor is added longitudinally to the 96-well microplate in a two-fold serial dilution using MHB, followed by two-fold serial dilutions of six times at concentrations ranging from 0.31 mg.mL$^{-1}$ to 10 mg.mL$^{-1}$. Using 0.5 Mcfarland turbidity as the quantitative standard for bacterial solution (0.5 Mcfarland = $10^8$ CFU/mL). Then, the concentration of the bacterial solution is diluted to 5 x $10^5$ CFU/mL, and 100 $\mu$L of the diluted bacterial solution is added to a 96-well microplate. Finally, the 96-well microplate is placed in a 37°C incubator for 18-24 hours. The bacteriostatic effects of $K.$ $pneumoniae$ 700623 and $K.$ $pneumoniae$ BAA-1705 in combination with different concentrations of Gentamicin and green mediated AgNP inhibitor are interpreted.

According to the Clinical and Laboratory Standards Institute (CLSI) standard, an FIC index less than or equal to 0.5 indicates synergy, an FIC index between 0.5-2 indicates indifference, and an FIC index greater than 2 indicates antagonism. The FIC index formula is as follows:

$$FIC = FICA + FICB$$

$$FICA = MIC \text{ concentration of A in combination / MIC concentration of A when used alone}$$

$$FICB = MIC \text{ concentration of B in combination / MIC concentration of B when used alone}$$

1. Synergistic assessment of $K.$ $pneumoniae$ 700623 (this experiment are performed at least in triplicate):

The growth of the bacteria is determined from the absorbance value, and the experimental results are shown in Table 1. For $K.$ $pneumoniae$ 700623, the MIC of Gentamicin when used alone is 40 $\mu$g.mL$^{-1}$, indicated by MIC$_G$; the MIC of the green mediated AgNP inhibitor when used alone is 2.5 mg.mL$^{-1}$, indicated by MIC$_M$. When used in combination, the concentration of Gentamicin is 5 $\mu$g.mL$^{-1}$, and the concentration of green mediated AgNP inhibitor is 0.63 mg.mL$^{-1}$, which is indicated by G-MIC-com and M-MIC-com, respectively. The FIC index is calculated to be 0.377 by the fractional inhibitory concentration (FIC) index formula, and it is confirmed that $K.$ $pneumoniae$ 70062 does not grow after 24 hours of spreading plate. The FIC index value of less than 0.5 indicates synergy when Gentamicin is combined with green mediated AgNP inhibitor.

Table 1

| Indication | $\mu g.mL^{-1}$ |
|---|---|
| $MIC_G$ | 40 |
| $MIC_M$ | 2.5 |
| G-MIC-com | 5 |
| M-MIC-com | 0.63 |

2. Synergistic assessment of *K. pneumoniae* BAA-1705 (this experiment are performed at least in triplicate):

The growth of the bacteria is determined from the absorbance value, and the experimental results are shown in Table 2. For *K. pneumoniae* BAA-1705, the MIC of Gentamicin when used alone is 40 $\mu g.mL^{-1}$, indicated by $MIC_G$; the MIC of the green mediated AgNP inhibitor when used alone is 5 mg.mL$^{-1}$, indicated by $MIC_M$. When used in combination, the concentration of Gentamicin is 10 $\mu g.mL^{-1}$, and the concentration of green mediated AgNP inhibitor is 0.63 mg.mL$^{-1}$, which is indicated by G-MIC-com and M-MIC-com, respectively. The FIC index is calculated to be 0.376 by the fractional inhibitory concentration (FIC) index formula, and it is confirmed that *K. pneumoniae* BAA-1705 does not grow after 24 hours of spreading plate. The FIC index value of less than 0.5 indicates synergy when Gentamicin is combined with green mediated AgNP inhibitor.

Table 2

| Indication | $\mu g.mL^{-1}$ |
|---|---|
| $MIC_G$ | 40 |
| $MIC_M$ | 5 |
| G-MIC-com | 10 |
| M-MIC-com | 0.63 |

The bioactivator of another embodiment may be one selected from a group consisting of an antibacterial agent, an antiviral agent, an antitumor agent, an anti-inflammatory agent, an analgesic agent, an anesthetic agent, a tissue regenerating agent and a combination thereof.

Conclusion: The synergistic antibacterial test is carried out with the silver-containing antibacterial colloid of the second embodiment of the present invention and Gentamicin, and the FIC index value is less than 0.5, indicating synergy when the two are combined. The concentration of Gentamicin can be greatly reduced to achieve its intended inhibition of pathogenic bacteria and reduce side effects during use.

Embodiments

1. A method for manufacturing an antibacterial colloid, comprising steps of: providing and mixing raw materials or precursors thereof constituting a ceramic substrate, a metal raw material or a precursor thereof, and a template surfactant of forming a mesoporous structure to form a mixture, wherein the raw materials or the precursors thereof constituting the ceramic substrate comprise at least silicon and oxygen; synthesizing the mixture to form an initial gel by a sol-gel technique; providing a three-dimensional configuration template, wherein the three-dimensional configuration template has a macroporous structure; immersing the three-dimensional configuration template in the initial gel at least one time; forming the ceramic substrate by performing a heat treatment at or above 400°C on the immersed three-dimensional configuration template, wherein the ceramic substrate has a hierarchically meso-macroporous structure having a plurality of first metal nanoparticles confined therein, and the plurality of first metal nanoparticles have a positive slow release effect; providing a medium, wherein the medium comprises a protein component containing at least a functional group for reduction; mixing and oscillating the medium with the ceramic substrate to cause the plurality of first metal nanoparticles to release a plurality of metal ions therefrom by the positive slow release effect; and reducing a part of the plurality of metal ions to nucleate and grow by the protein component, wherein the part of the plurality of metal ions grow up to form a plurality of second metal nanoparticles, wherein the antibacterial colloid comprises the medium, the plurality of second metal nanoparticles having an average particle diameter less than 10 nm, and the plurality of metal ions having a concentration greater than 20 ppm, and the antibacterial colloid is free from nitrate ions.

2. The method of Embodiment 1, further comprising a step of providing a filter to filtrate the antibacterial colloid to remove the ceramic substrate and impurities.

3. The method of Embodiments 1-2, wherein the hierarchically meso-macroporous structure comprises a plurality of macropores and a wall having a plurality of arranged mesopores, and the plurality of macropores are separated by the wall.

4. The method of Embodiments 1-3, wherein the wall is formed from the raw materials or the precursors thereof, and the plurality of first metal nanoparticles are formed from the metal raw material or the precursor thereof.

5. The method of Embodiments 1-4, wherein the template surfactant of forming the mesoporous structure and the immersed three-dimensional configuration template are removed during the heat treatment, the macroporous structure provides channels for removing the template surfactant of forming the mesoporous structure and the immersed three-dimensional configuration template.

6. The method of Embodiments 1-5, wherein the protein component is casein.

7. The method of Embodiments 1-6, wherein the casein is obtained from animal milk or a plant extract.

8. The method of Embodiments 1-7, wherein the casein has a concentration ranging from 10 g/L to 30 g/L.

9. The method of Embodiments 1-8, wherein when a total quantity of the raw materials or the precursors thereof constituting the ceramic substrate is $M_1$ mole, a quantity of the silicon included in the raw materials or the precursors thereof constituting the ceramic substrate is $M_{Si}$ mole, a quantity of the metal raw material or the precursor thereof is $M_{metal}$ mole, the $M_{Si}$ is at least 70% of the $M_1$, and the $M_{metal}$ is less than or equal to 10% of the $M_1$.

10. The method of Embodiments 1-9, wherein the $M_{metal}$ is 1% of the $M_1$.

11. The method of Embodiments 1-10, wherein a metal in the metal raw material or the precursor thereof is one selected from a group consisting of gold, silver, strontium, zinc, copper, iron and a combination thereof.

12. The method of Embodiments 1-11, wherein the antibacterial colloid has a property of inhibiting the growth of a microorganism or killing a microorganism. The microorganism may be a bacterium, a virus, a fungus or a protozoan, and the bacterium may be one selected from a group consisting of *Staphylococcus aureus*, *Pseudomonas aeruginosa*, Methicillin-resistant *staphylococcus aureus*, *Klebsiella pneumoniae* and a combination thereof.

13. The method of Embodiments 1-12, wherein the plurality of second metal nanoparticles have an average particle diameter ranging from 2 nm to 5 nm.

14. The method of Embodiments 1-13, wherein one of the raw materials or the precursors thereof constituting the ceramic substrate is Tetraethyl orthosilicate; the metal raw material or the precursor thereof is silver nitrate, gold hydrogen nitrate, zinc nitrate hexahydrate, cupric nitrate trihydrate, iron trinitrate nonahydrate or strontium nitrate; and the template surfactant of forming the mesoporous structure is a thermoreversible hydrogel (Pluronic F-127).

15. The method of Embodiments 1-14, wherein the three-dimensional configuration template is a porous organism or a synthetic porous body.

16. The method of Embodiments 1-15, wherein the porous organism is a natural sponge, and the synthetic porous body is a polyurethane foam or a polylactic acid macroporous structure.

17. The method of Embodiments 1-16, wherein a condition of oscillating is shaking at 160 rpm for 24 hours at 35 °C.

18. The method of Embodiments 1-17, wherein the functional group for reduction is S-H or N-H.

19. An antibacterial colloid in a system containing microorganisms, comprising: a plurality of metal nanoparticles, wherein the plurality of metal nanoparticles have an average particle diameter less than 10 nm; a plurality of metal ions, wherein the plurality of metal ions have a concentration greater than 20 ppm; and a medium, wherein the medium comprises a protein component containing at least a functional group for reduction, wherein: the antibacterial colloid is free from nitrate ions; and the microorganisms have a first value A1 of the colony forming unit (CFU) in a first state, and a second value A2 of the CFU in a second state after the antibacterial colloid is added to the system for a specific period of time where (A1-A2)/A1 is greater than or equal to 0.5.

20. The antibacterial colloid of Embodiment 19, wherein the microorganism is one selected from a group consisting of *Staphylococcus aureus*, *Pseudomonas aeruginosa*, Methicillin-resistant *staphylococcus aureus*, *Klebsiella pneumoniae* and a combination thereof.

21. The antibacterial colloid of Embodiments 19-20, wherein the plurality of metal nanoparticles have an average particle diameter ranging from 2 nm to 5 nm.

22. The antibacterial colloid of Embodiments 19-21, wherein the functional group for reduction is S-H or N-H.

23. The antibacterial colloid of Embodiments 19-22, wherein the protein component is casein.

24. The antibacterial colloid of Embodiments 19-23, wherein the protein component has a concentration ranging from 10 g/L to 30 g/L.

25. The antibacterial colloid of Embodiments 19-24, wherein the system is a cell, a biological tissue, an organ, a cosmetic, a medicine, a medical appliance, or a biomaterial.

26. A system comprising microorganisms, wherein adding a bioactivator and an antibacterial colloid in the system, after a specific period of time, an antimicrobial effect produces, and the system has a fractional inhibitory concentration (FIC) index, and the antibacterial colloid comprises a plurality of metal nanoparticles, wherein the plurality of metal nanoparticles have an average particle diameter less than 10 nm; a plurality of metal ions, wherein the plurality of metal ions have a concentration greater than 20 ppm; and a medium, wherein the medium comprises a protein component containing at least a functional group for reduction, wherein the antibacterial colloid is free from nitrate ions, and the FIC index is less than or equal to 0.5.

27. The system of Embodiment 26, wherein the bioactivator is Gentamicin.

28. The system of Embodiments 26-27, wherein the microorganisms are one selected from a group consisting of *Staphylococcus aureus, Pseudomonas aeruginosa,* Methicillin-resistant *staphylococcus aureus, Klebsiella pneumoniae* and a combination thereof.

29. The system of Embodiments 26-28, wherein the plurality of metal nanoparticles have an average particle diameter ranging from 2 nm to 5 nm.

30. The system of Embodiments 26-29, wherein the functional group for reduction is S-H or N-H.

31. The system of Embodiments 26-30, wherein the protein component is casein.

32. The system of Embodiments 26-31, wherein the protein component has a concentration ranging from 10 g/L to 30 g/L.

33. An antibacterial colloid, comprising: a plurality of metal nanoparticles, wherein the plurality of metal nanoparticles have an average particle diameter less than 10 nm; a plurality of metal ions, wherein the plurality of metal ions have a concentration greater than 20 ppm; and a medium, wherein the medium comprises a protein component containing at least a functional group for reduction, wherein the antibacterial colloid is free from nitrate ions.

34. The antibacterial colloid of Embodiment 33, further comprising a ceramic substrate.

35. The antibacterial colloid of Embodiments 33-34, wherein the protein component is obtained from an animal or a plant.

36. The antibacterial colloid of Embodiments 33-35, wherein the protein component is casein.

37. The antibacterial colloid of Embodiments 33-36, wherein the casein is obtained from animal milk or a plant extract.

38. The antibacterial colloid of Embodiments 33-37, wherein the casein has a concentration ranging from 10 g/L to 30 g/L.

39. The antibacterial colloid of Embodiments 33-38, wherein the plurality of metal ions have a concentration greater than 40 ppm.

40. The antibacterial colloid of Embodiments 33-39, wherein the plurality of metal nanoparticles have an average particle diameter ranging from 2 nm to 5 nm.

41. The antibacterial colloid of Embodiments 33-40, wherein the antibacterial colloid has a property of inhibiting the growth of a microorganism or killing a microorganism, and the microorganism is one selected from a group consisting of *Staphylococcus aureus*, *Pseudomonas aeruginosa*, Methicillin-resistant *staphylococcus aureus*, *Klebsiella pneumoniae* and a combination thereof.

42. The antibacterial colloid of Embodiments 33-41, wherein the functional group for reduction is S-H or N-H.

## Claims

1. A method for manufacturing an antibacterial colloid, **characterized by** comprising steps of:

   providing and mixing raw materials or precursors thereof constituting a ceramic substrate, a metal raw material or a precursor thereof, and a template surfactant of forming a mesoporous structure to form a mixture, wherein the raw materials or the precursors thereof constituting the ceramic substrate comprise at least silicon and oxygen;
   synthesizing the mixture to form an initial gel by a sol-gel technique;
   providing a three-dimensional configuration template, wherein the three-dimensional configuration template has a macroporous structure;
   immersing the three-dimensional configuration template in the initial gel at least one time;
   forming the ceramic substrate by performing a heat treatment at or above 400°C on the immersed three-dimensional configuration template, wherein the ceramic substrate has a hierarchically meso-macroporous structure having a plurality of first metal nanoparticles confined therein, and the plurality of first metal nanoparticles have a positive slow release effect;
   providing a medium, wherein the medium comprises a protein component containing at least a functional group for reduction;

mixing and oscillating the medium with the ceramic substrate to cause the plurality of first metal nanoparticles to release a plurality of metal ions therefrom by the positive slow release effect; and

reducing a part of the plurality of metal ions to nucleate and grow by the protein component, wherein the part of the plurality of metal ions grow up to form a plurality of second metal nanoparticles;

wherein the antibacterial colloid comprises the medium, the plurality of second metal nanoparticles having an average particle diameter less than 10 nm, and the plurality of metal ions having a concentration greater than 20 ppm, and the antibacterial colloid is free from nitrate ions.

2. The method according to Claim 1, **characterized by** further comprising a step of:
providing a filter to filtrate the antibacterial colloid to remove the ceramic substrate and impurities.

3. The method according to Claims 1 or 2, **characterized in that** the hierarchically meso-macroporous structure comprises a plurality of macropores and a wall having a plurality of arranged mesopores, and the plurality of macropores are separated by the wall.

4. The method according to Claim 3, **characterized in that** the wall is formed from the raw materials or the precursors thereof constituting the ceramic substrate, and the plurality of first metal nanoparticles are formed from the metal raw material or the precursor thereof.

5. The method according to any one of Claims 1-4, **characterized in that** the template surfactant of forming the mesoporous structure and the immersed three-dimensional configuration template are removed during the heat treatment, the macroporous structure provides channels for removing the template surfactant of forming the mesoporous structure and the immersed three-dimensional configuration template.

6. The method according to any one of Claims 1-5, **characterized in that** when a total quantity of the raw materials or the precursors thereof constituting the ceramic substrate is $M_1$ mole, a quantity of the silicon included in the raw materials or the precursors thereof constituting the ceramic substrate is $M_{Si}$ mole, a quantity of the metal raw material or the precursor thereof is $M_{metal}$ mole, the $M_{Si}$ is at least 70% of the $M_1$, and the $M_{metal}$ is less than or equal to 10% of the $M_1$.

7. The method according to Claim 6, **characterized in that** the $M_{metal}$ is 1% of the $M_1$.

8. The method according to any one of Claims 1-7, **characterized in that** a metal in the metal raw material or the precursor thereof is one selected from a group consisting of gold, silver, strontium, zinc, copper, iron and a combination thereof.

9. An antibacterial colloid, **characterized by** comprising:

a plurality of metal nanoparticles, wherein the plurality of metal nanoparticles have an average particle diameter less than 10 nm;

a plurality of metal ions, wherein the plurality of metal ions have a concentration greater than 20 ppm; and

a medium, wherein the medium comprises a protein component containing at least a functional group for reduction,

wherein the antibacterial colloid is free from nitrate ions.

10. The antibacterial colloid according to Claim 9, **characterized by** further comprising a ceramic substrate.

11. The antibacterial colloid according to Claims 9 or 10, **characterized in that** the protein component is obtained from an animal or a plant.

12. The antibacterial colloid according to any one of Claims 9-11, **characterized in that** the protein component is casein.

13. The antibacterial colloid according to Claim 12, **characterized in that** the casein is obtained from animal milk or a plant extract.

14. The antibacterial colloid according to Claims 12 or 13, **characterized in that** the casein has a concentration ranging from 10 g/L to 30 g/L.

**15.** The antibacterial colloid according to any one of Claims 9-14, **characterized in that** the plurality of metal ions have the concentration greater than 40 ppm.

EP 3 714 692 A1

```
┌──────────────────┐
│ Forming a        │  11
│ silver-containing │
│ ceramic          │
│ substrate having │
│ a hierarchically │
│ meso-macroporous │
│ structure        │
└──────────────────┘
```

10

```
                    ┌──────────────────┐   ┌──────────────┐   ┌──────────────────┐   ┌──────────────────┐
                    │ Stirring and     │13 │ Silver ions  │14 │ Using a filter   │15 │ Silver-containing │16
                    │ mixing the       │   │ reduce,      │   │ to remove the    │   │ antibacterial    │
                    │ silver-containing │   │ nucleate,    │   │ silver-containing │   │ colloid          │
                    │ ceramic          │   │ and grow to  │   │ ceramic          │   │                  │
                    │ substrate        │   │ re-form      │   │ substrate having │   │                  │
                    │ with the medium  │   │ silver       │   │ the hierarchically│   │                  │
                    │ comprising the   │   │ nanoparticles │   │ meso-macroporous │   │                  │
                    │ protein          │   │              │   │ structure and    │   │                  │
                    │ component        │   │              │   │ other impurities │   │                  │
                    └──────────────────┘   └──────────────┘   └──────────────────┘   └──────────────────┘
```

```
┌──────────────────┐
│ Providing a      │
│ medium comprising │
│ a protein        │
│ component        │
└──────────────────┘
        12
```

Fig. 1

20 nm

Fig. 2

Fig. 3

Fig. 4

Fig. 5

Fig. 6

Fig. 7

Fig. 8

Fig. 9

Fig.10

Fig.11

Europäisches
Patentamt

European
Patent Office

Office européen
des brevets

# EUROPEAN SEARCH REPORT

**Application Number**

EP 20 16 6237

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/368416 A1 (SHIH CHI-JEN [TW] ET AL) 27 December 2018 (2018-12-27)<br>* paragraphs [0041], [0049], [0050]; claim 13 *<br>* the whole document * | 1-15 | INV.<br>A01N25/08<br>C04B35/14<br>C04B35/624 |
| A | DATABASE WPI<br>Week 201702<br>Thomson Scientific, London, GB;<br>AN 2016-70784E<br>XP002800054,<br>-& RU 2 602 741 C2 (UNIV TOMSK POLYTECHNIC) 20 November 2016 (2016-11-20)<br>* abstract *<br>* citations refer to the machine translation into English;<br>paragraphs [0011] - [0015] *<br>* the whole document * | 1-15 | |
| A | MARIBEL GUZMAN ET AL: "Synthesis and antibacterial activity of silver nanoparticles against gram-positive and gram-negative bacteria",<br>NANOMEDICINE: NANOTECHNOLOGY, BIOLOGY AND MEDICINE, ELSEVIER, NL,<br>vol. 8, no. 1, 4 May 2011 (2011-05-04),<br>pages 37-45, XP028122783,<br>ISSN: 1549-9634, DOI:<br>10.1016/J.NANO.2011.05.007<br>[retrieved on 2011-06-02]<br>* page 42, left-hand column - page 42, right-hand column, paragraph 1 *<br>* the whole document * | 1-15 | TECHNICAL FIELDS SEARCHED (IPC)<br><br>A01N<br>C04B |

-/--

The present search report has been drawn up for all claims

| Place of search | Date of completion of the search | Examiner |
|---|---|---|
| The Hague | 17 August 2020 | Straub, Thomas |

Europäisches
Patentamt

European
Patent Office

Office européen
des brevets

## EUROPEAN SEARCH REPORT

Application Number

EP 20 16 6237

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE WPI<br>Week 201122<br>Thomson Scientific, London, GB;<br>AN 2010-J71847<br>XP002800055,<br>-& RU 2008 140578 A (UNIV SARAT)<br>20 April 2010 (2010-04-20)<br>* abstract; claim 1 *<br>* citations refer to the machine translation into English. *<br>* the whole document *<br>----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 17 August 2020 | Straub, Thomas |

EPO FORM 1503 03.82 (P04C01)

## ANNEX TO THE EUROPEAN SEARCH REPORT
## ON EUROPEAN PATENT APPLICATION NO.

EP 20 16 6237

This annex lists the patent family members relating to the patent documents cited in the above-mentioned European search report.
The members are as contained in the European Patent Office EDP file on
The European Patent Office is in no way liable for these particulars which are merely given for the purpose of information.

17-08-2020

| Patent document cited in search report | | Publication date | Patent family member(s) | | Publication date |
|---|---|---|---|---|---|
| US 2018368416 | A1 | 27-12-2018 | CN 109111178 A | | 01-01-2019 |
| | | | TW 201904912 A | | 01-02-2019 |
| | | | US 2018368416 A1 | | 27-12-2018 |
| | | | US 2020113185 A1 | | 16-04-2020 |
| RU 2602741 | C2 | 20-11-2016 | NONE | | |
| RU 2008140578 | A | 20-04-2010 | NONE | | |

EPO FORM P0459

For more details about this annex : see Official Journal of the European Patent Office, No. 12/82